# EUROPEAN PATENT APPLICATION

(11) **EP 4 610 630 A1**
(43) Date of publication of application: **03.09.2025**
(21) Application number: 23882869.3
(22) Date of filing: 28.08.2023
(51) Int. Cl.: G01N 21/78, G01N 1/40, G01N 33/44

(54) **AUTOMATIC ANALYSIS METHOD AND AUTOMATIC ANALYSIS SYSTEM FOR POLYMER END GROUPS**

(30) Priority: 25.10.2022 KR 20220138598
(71) Applicant: SK Chemicals Co., Ltd., Seongnam-si, Gyeonggi-do 13494 (KR)
(72) Inventor: KIM, A-Hyun, Seongnam-si, Gyeonggi-do 13494 (KR); HAN, Min-Young, Seongnam-si, Gyeonggi-do 13494 (KR)
(74) Representative: Dehns
(86) International application number: PCT/KR2023/012699
(87) International publication number: WO 2024/090762

(57) **Abstract**

The present invention relates to an automatic analysis method and an automatic analysis system for polymer end groups. As an analysis process following the weighing of a polymer sample is performed automatically, the present invention may provide a tester with an improved analysis working environment and acquire highly reliable analysis results.

## Description

### Technical Field

The present invention relates to an automatic analysis method and an automatic analysis system for polymer end groups that can predict the molecular weight and characteristics (physical properties) of a polymer by analyzing the end groups of the polymer through an automated process.

### Background Art

Analysis of end groups bound to a polymer is used as one of the methods to predict the molecular weight of the polymer and to identify basic characteristics that affect its physical properties after processing the polymer.

The method of analyzing the end groups of a polymer includes titrating a polymer sample. Specifically, a polymer sample is weighed and then dissolved and cooled, and an indicator and a titrant are added to visually observe the change in color to analyze the content of end groups of the polymer.

However, the method of titrating a polymer sample has the limitation of being labor-intensive since the entire procedure is performed manually. There is also a problem in that errors occur in analysis results due to differences in determination standards between testers. In addition, the tester is exposed to high temperatures to dissolve the solvents and polymer samples used in each procedure; thus, the safety of the tester during testing may be impaired. In addition, there is also the inconvenience of having to wash all test tubes, beakers, and other equipment used in the titration procedure in order to reuse them.

Thus, there is a need to develop analysis techniques in analyzing polymer end groups that can improve the tester's working environment while reducing errors in analysis results.

### [Prior Art Document]

### [Patent Document]

(Patent Document 1) Korean Laid-open Patent Publication No. 2020-0046622

### Disclosure of Invention

### Technical Problem

An object of the present invention is to provide an automatic analysis method and an automatic analysis system for polymer end groups that can minimize errors in analysis results and improve the tester's work environment by analyzing the end groups of the polymer through an automated process.

### Solution to Problem

In order to accomplish the above object, the present invention provides a method for automatic analysis of polymer end groups, which comprises (1) mounting a container containing a polymer sample into an auto sampler; (2) adding a solvent comprising an aromatic compound having at least one hydroxy group into the container containing the polymer sample and heating it to obtain a polymer sample solution; (3) cooling the polymer sample solution; (4) adding an indicator to the cooled polymer sample solution and titrating it with a titrant to observe the change in the potential value of a colorimetric electrode; and (5) analyzing the change in the potential value through software to calculate the content of polymer end groups.

In addition, the present invention provides a system for automatic analysis of polymer end groups, which comprises a sample supply unit comprising an auto sampler into which a container containing a polymer sample is mounted; a heating unit comprising a solvent injection tube for injecting a solvent comprising an aromatic compound having at least one hydroxy group into the container containing the polymer sample, a heating rod for heating the polymer sample injected with the solvent, and a cooling solvent injection tube for injecting a cooling solvent to cool the polymer sample solution obtained through the heating; a titration unit comprising an indicator injection tube for injecting an indicator into the polymer sample solution cooled through the cooling solvent, a titrant injection tube for injecting a titrant into the polymer sample solution in which the indicator has been injected, and a colorimetric electrode that shows a change in the potential value depending on the amount of the titrant injected; a dispense control unit comprising a solvent dispenser for dispensing the solvent into the solvent injection tube, an indicator dispenser for dispensing the indicator into the indicator injection tube, and a titrant dispenser for dispensing the titrant into the titrant injection tube; a pump control unit comprising a solvent supply pump for supplying the solvent to the solvent dispenser and a cooling solvent supply pump for supplying the cooling solvent to the cooling solvent injection tube; and an automatic data processing unit comprising software for analyzing the change in the potential value and an output device for outputting analysis results obtained through the software.

### Advantageous Effects of Invention

According to the present invention, since the analysis procedure of polymer end groups is controlled through software, highly accurate analysis results can be obtained. In addition, since the entire procedure of analyzing end groups after weighing a polymer sample is carried out automatically, the work environment (ensuring safety and reducing labor) for the tester (analyst) can be improved.

### Brief Description of Drawings

Fig. 1 is a flowchart showing the automatic analysis procedure of polymer end groups according to an embodiment of the present invention.
Fig. 2 is a schematic diagram showing the automatic analysis system for polymer end groups according to an embodiment of the present invention.

### Best Mode for Carrying out the Invention

Hereinafter, the present invention will be described in detail. The present invention herein is not limited to the disclosures given below, but it may be modified into various forms as long as the gist of the invention is not changed.

In the present specification, the term "comprising" is intended to specify a particular characteristic, region, step, process, element, and/or component. It does not exclude the presence or addition of any other characteristic, region, step, process, element and/or component, unless specifically stated to the contrary.

All numbers and expressions related to the quantities of components, reaction conditions, and the like used herein are to be understood as being modified by the term "about" unless otherwise indicated.

For the sake of description, the sizes of individual elements in the appended drawings may be exaggeratedly depicted, and they may differ from the actual sizes.

### Method for automatic analysis of polymer end groups

The present invention relates to a method for analyzing the end groups of a polymer to predict the molecular weight of the polymer and to identify basic characteristics that affect its physical properties after processing the polymer. It is characterized in that the entire procedure after weighing a polymer sample is carried out automatically.

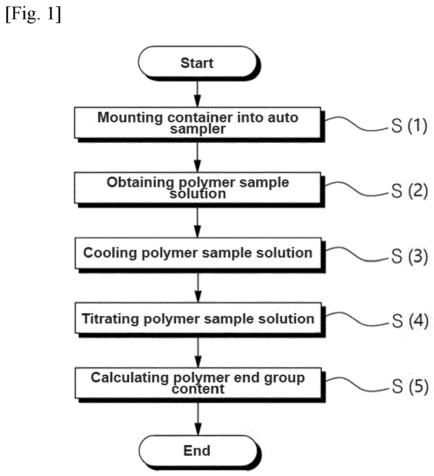

Specifically, the method for automatic analysis of polymer end groups according to the present invention comprises (1) mounting a container containing a polymer sample into an auto sampler; (2) adding a solvent comprising an aromatic compound having at least one hydroxy group into the container containing the polymer sample and heating it to obtain a polymer sample solution; (3) cooling the polymer sample solution; (4) adding an indicator to the cooled polymer sample solution and titrating it with a titrant to check the change in the potential value of a colorimetric electrode; and (5) analyzing the change in the potential value through software to calculate the content of polymer end groups. Hereinafter, this will be described by referring to Fig. 1.

### Step (1): Mounting a container into an auto sampler

According to the present invention, step (1) is to mount a container containing a polymer sample into an auto sampler. The number of containers mounted into the auto sampler may be one or more. Specifically, it may be 1 to 25, 2 to 20, or 5 to 15.

Meanwhile, the polymer sample may be weighed in a predetermined amount before being placed in the container.

### Step (2): Obtaining a polymer sample solution

According to the present invention, step (2) is to add a solvent comprising an aromatic compound having at least one hydroxy group into the container containing the polymer sample and heating it to obtain a polymer sample solution.

The aromatic compound having at least one hydroxy group (specifically, 1 to 4 hydroxy groups or 1 to 2 hydroxy groups) and contained in the solvent is not particularly limited as long as it is a compound that does not react with the polymer sample and does not have a carboxyl group. Specifically, the aromatic compound having at least one hydroxy group may comprise at least one selected from the group consisting of phenol, o-cresol, m-cresol, p-cresol, o-chlorophenol, m-chlorophenol, p-chlorophenol, 2,6-xylenol, catechol, resorcinol, hydroquinone, pyrogallol, and benzyl alcohol. As the solvent comprises the aromatic compound, the polymer sample can be dissolved at a relatively low heating temperature, thereby stabilizing the heating procedure (for example, the procedure of heating a mixture of a polymer sample and a solvent with a heating rod). As a result, automation of the analysis procedure can be implemented.

Heating of the polymer sample into which the solvent has been injected may be specifically carried out at 60 to 100°C for 400 to 900 seconds. More specifically, the heating may be carried out at 60 to 90°C, 60 to 85°C, 63 to 80°C, 65 to 75°C, 65 to 70°C, or 65 to 68°C for 400 to 800 seconds, 430 to 700 seconds, 450 to 650 seconds, 500 to 650 seconds, 500 to 600 seconds, or 500 to 550 seconds. As the heating temperature and heating time are within the above ranges, the polymer sample is well dissolved without the solvent boiling over, and the polymer sample solution can be efficiently obtained.

Meanwhile, the solvent comprising the aromatic compound having at least one hydroxy group has a low freezing point; thus, when the temperature of the analysis environment falls below a certain level, it may freeze and stick to the container. Thus, the solvent may further comprise at least one auxiliary solvent selected from the group consisting of chloroform, dichloromethane, and isopropyl alcohol. As the solvent further comprises the auxiliary solvent, the freezing point of the solvent can be raised to prevent the solvent from freezing (preventing solvent precipitation). As a result, automation of the analysis procedure can be well implemented.

When the solvent comprises the aromatic compound having at least one hydroxy group (a) and the auxiliary solvent (b), their mixing ratio (a:b) is not particularly limited. Specifically, it may be a weight ratio of 5:1 to 1:5, a weight ratio of 4:1 to 1:4, a weight ratio of 3:1 to 1:3, or a weight ratio of 2:1 to 1:2. As the mixing ratio is within the above range, the solvent does not freeze, and the polymer sample with the solvent is well dissolved within the heating temperature and heating time ranges, whereby the polymer sample solution can be efficiently obtained.

### Step (3): Cooling the polymer sample solution

According to the present invention, step (3) is to cool the polymer sample solution. Cooling of the polymer sample solution may be carried out naturally or intentionally. Specifically, the natural cooling may be carried out by leaving the polymer sample solution at room temperature for a certain period of time. The intentional cooling may be carried out by injecting a cooling solvent into the polymer sample solution.

The cooling solvent may comprise at least one selected from the group consisting of chloroform, dichloromethane, and isopropyl alcohol. As the polymer sample solution is cooled by the cooling solvent, the polymer sample solution can be cooled in a short period of time. In addition, in the procedure of injecting the cooling solvent into the polymer sample solution, the heating rod used to heat the mixture of the polymer sample and the solvent may be automatically washed. Since the need for a manual washing procedure to manage the heating rod as a heating source is eliminated as a result, the present invention can provide an improved work environment to the tester.

### Step (4): Titration of the polymer sample solution

According to the present invention, step (4) is to add an indicator to the cooled polymer sample solution and titrate it with a titrant to observe the change in the potential value of a colorimetric electrode. Conditions such as addition amount, addition speed, and addition time of each of the indicator and the titrant to the polymer sample solution may be automatically controlled through values preset (input) in the software. In addition, the change in the potential value of the colorimetric electrode can also be checked by the output of results collected by the software.

The indicator may specifically comprise at least one selected from the group consisting of bromophenol blue, phenol red, and methyl red. As the indicator comprises the above component, the sensitivity (reactivity) of the colorimetric electrode increases, allowing highly accurate analysis results to be obtained.

The titrant may specifically comprise at least one selected from the group consisting of sodium hydroxide (NaOH) and potassium hydroxide (KOH). As the titrant comprises the above component, titration efficiency can be increased.

### Step (5): Calculation of the content of polymer end groups

According to the present invention, step (5) is to analyze the change in the potential value through software to calculate the content of polymer end groups. Specifically, the software analyzes the inflection point (equivalence point change) in the data in which the change in the potential value is collected to automatically calculate the content of polymer end groups.

The software may be integrated with the software that controls the conditions for adding the indicator and titrant **in** step (4) or may be a separate one.

Meanwhile, the method for automatic analysis of polymer end groups according to the present invention may further comprise a step (step (6)) of washing the colorimetric electrode of step (4) by supplying a washing solvent. The washing solvent may specifically be at least one selected from the group consisting of acetone, acetonitrile, isopropanol, and water. As the washing solvent comprises the above component, the colorimetric electrode can be washed efficiently. Since the need for a manual washing procedure to manage the colorimetric electrode is eliminated as a result, the present invention can provide an improved work environment to the tester.

The polymer (polymer sample) that can be analyzed by the automatic analysis method according to the present invention is not particularly limited as long as it is a polymer having a carboxyl group. Specifically, it may be polyethylene terephthalate (PET) or polyethylene terephthalate glycol (PETG).

In addition, the end group of the polymer, which is the subject of analysis by the automatic analysis method according to the present invention, is not particularly limited. It may be a carboxyl group (COOH) whose content can be relatively accurately confirmed by the automatic analysis method.

As described above, as the automatic analysis method according to the present invention automatically performs the procedures such as dissolution, cooling, titration, and washing after weighing a polymer sample, the labor input into the analysis procedure can be significantly reduced while ensuring the stability of the tester's analysis work. In addition, since the analysis procedure of polymer end groups is controlled through software in the automatic analysis method according to the present invention, highly accurate analysis results can be obtained.

### System for automatic analysis of polymer end groups

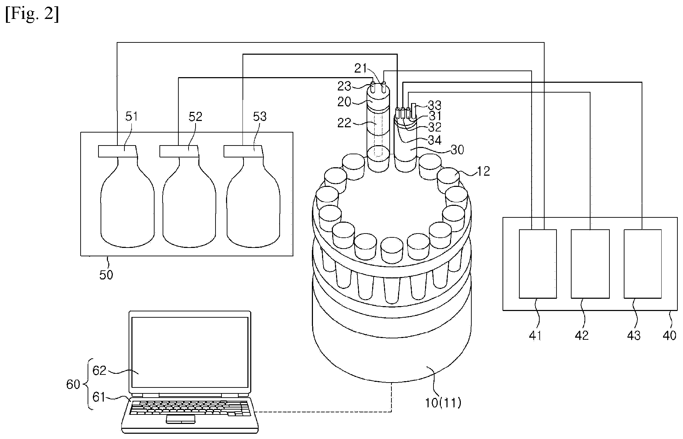

The present invention provides a system for automatic analysis of polymer end groups in which the above automatic analysis method can be implemented. Specifically, the system for automatic analysis of polymer end groups according to the present invention comprises a sample supply unit; a heating unit; a titration unit; a dispense control unit; a pump control unit; and an automatic data processing unit. Hereinafter, this will be described by referring to Fig. 2.

### Sample supply unit

According to the present invention, the sample supply unit (10) comprises an auto sampler (11) into which a container (12) containing a polymer sample is mounted.

The auto sampler (11) sequentially supplies the container (12) containing a polymer sample to a heating unit (20) and a titration unit (30). At least one container (12), specifically 1 to 25, 2 to 20, or 5 to 15 containers may be mounted. As the auto sampler (11) into which a plurality of containers (12) can be mounted is used, and the mounted containers (12) can be automatically supplied to the heating unit (20) and the titration unit (30), where heating and titration procedures are carried out, respectively, multiple polymer samples can be analyzed at once to obtain highly accurate analysis results in the present invention.

Meanwhile, the container (12) to be mounted into the auto sampler (11) may be a disposable container or a multi-use container. Specifically, it may be a disposable container made of polypropylene (PP). As the container (12) is a disposable container, the conventional operation of washing the used containers upon completion of analysis can be omitted. Here, the use of a disposable container may be possible since heating in the heating unit (20) is performed at a relatively low temperature (e.g., 60 to 90°C).

### Heating unit

According to the present invention, the heating unit (20) comprises a solvent injection tube (21) for injecting a solvent comprising an aromatic compound having at least one hydroxy group into the container (12) containing a polymer sample; a heating rod (22) for heating the polymer sample injected with the solvent; and a cooling solvent injection tube (23) for injecting a cooling solvent to cool the polymer sample solution obtained through the heating

The solvent injection tube (21) and the cooling solvent injection tube (23) may not be particularly limited as long as they each have a structure that allows the solvent and the cooling solvent to flow smoothly and are made of a material that is durable against each solvent. In addition, the heating rod (22) may not be particularly limited as long as it can stably dissolve the polymer sample into which the solvent has been injected.

Here, for stable operation of the heating rod (22), the solvent injected through the solvent injection tube (21) may further comprise at least one auxiliary solvent selected from the group consisting of chloroform, dichloromethane, and isopropyl alcohol. That is, a mixed solvent in which the aromatic compound having at least one hydroxy group and the auxiliary solvent are mixed may be injected into the container (12) through the solvent injection tube (21).

In addition, the heating rod (22) can be washed along with the cooling of the polymer sample solution by the cooling solvent injected through the cooling solvent injection tube (23). In such an event, the washing procedure of the heating rod (22) using the cooling solvent may be carried out simultaneously with the cooling procedure of the polymer sample solution or may be carried out before or after the cooling procedure of the polymer sample solution.

The heating unit (20) may further comprise a stirrer (not shown) to facilitate the dissolution of the polymer sample and a discharge device (not shown) to automatically discharge the cooling solvent used in the washing procedure of the heating rod (22).

### Titration unit

The titration unit (30) comprises an indicator injection tube (31) for injecting an indicator into the polymer sample solution cooled through the cooling solvent; a titrant injection tube (32) for injecting a titrant into the polymer sample solution in which the indicator has been injected; and a colorimetric electrode (33) that shows a change in the potential value depending on the amount of the titrant injected.

The indicator injection tube (31) and the titrant injection tube (32) may not be particularly limited as long as they each have a structure that allows the indicator and the titrant to flow smoothly and are made of a material that is durable against each of the indicator and the titrant.

In addition, the colorimetric electrode (33) may not be particularly limited as long as it clearly shows a change in the potential value (change in color) depending on the amount of the titrant injected. Specifically, the colorimetric electrode (33) may be a photometric sensor electrode (optrode). As the photometric sensor electrode is used, titration analysis can be carried out quickly, simply, and accurately.

Meanwhile, the titration unit (30) may further comprise a washing solvent injection tube (34) for injecting a washing solvent for washing the colorimetric electrode (33). The washing solvent injection tube (34) may not be particularly limited as long as it has a structure that allows the washing solvent to flow smoothly and is made of a material that is durable against the washing solvent.

In addition, the titration unit (30) may further comprise a stirrer (not shown) to facilitate the titration of the polymer sample solution and a discharge device (not shown) to automatically discharge the washing solvent used in the washing procedure of the colorimetric electrode (33).

### Dispense control unit

According to the present invention, the dispense control unit (40) comprises a solvent dispenser (41) for dispensing the solvent into the solvent injection tube (21), an indicator dispenser (42) for dispensing the indicator into the indicator injection tube (31), and a titrant dispenser (43) for dispensing the titrant into the titrant injection tube (32).

The solvent dispenser (41), the indicator dispenser (42), and the titrant dispenser (43) may not be particularly limited as long as they each have a structure that allows the solvent, the indicator, and the titrant to flow smoothly and are made of a material that is durable against each of the solvent, the indicator, and the titrant.

As the injection amount and injection speed of each of the solvent, the indicator, and the titrant are precisely controlled through the dispense control unit (40), the present invention allows the analysis procedure to be carried out efficiently and highly accurate analysis results to be obtained.

### Pump control unit

According to the present invention, the pump control unit (50) comprises a solvent supply pump (51) for supplying the solvent to the solvent dispenser (41); and a cooling solvent supply pump (52) for supplying the cooling solvent to the cooling solvent injection tube (23).

In addition, the pump control unit (50) may further comprise a washing solvent supply pump (53) for supplying the washing solvent to the washing solvent injection tube (34).

The solvent supply pump (51), the cooling solvent supply pump (52), and the washing solvent supply pump (53) may not be particularly limited as long as they are each a pump that allows each solvent to flow.

Since the pump control unit (50) controls the supply and flow of each solvent, the present invention can implement automation of the analysis procedure.

### Automatic data processing unit

According to the present invention, the automatic data processing unit (60) comprises software (61) for analyzing the change in the potential value and an output device (62) for outputting analysis results obtained through the software.

Specifically, the automatic data processing unit (60) may be a commonly known computer. The automatic data processing unit (60) is connected wired or wirelessly to each of the sample supply unit (10), the heating unit (20), the titration unit (30), the dispense control unit (40), and the pump control unit (50). Therefore, it is possible to set (control) the operating conditions that must be applied when each unit is operated.

Since the heating unit (20) and the titration unit (30) are separated in the automatic analysis system according to the present invention, heat is not applied to the colorimetric electrode (33) during the heating procedure, whereby the durability of the colorimetric electrode (33) may not be deteriorated by heat. In addition, the dissolution procedure, cooling procedure, and titration procedure of a polymer sample, along with the washing procedure of the used test equipment, are all performed automatically; thus, it is possible to provide testers with an optimized analysis environment and reliable analysis results.

### Mode for the Invention

Hereinafter, the present invention will be described in more detail with reference to embodiments. However, these examples are provided only for illustration purposes, and the present invention is not limited thereto.

### [Example 1]

A PET semidal chip was selected as a polymer sample, and the automatic analysis system shown in Fig. 2 was used to automatically analyze the content of carboxyl groups bonded as PET end groups. Specifically, PET semidal chips were placed in a disposable container made of polypropylene, which was mounted into an auto sampler.

Next, 60 ml of a mixed solvent containing o-cresol and chloroform at a weight ratio of 2:1 was injected into the disposable container, and the mixture was heated at 65°C for 500 seconds to obtain a PET solution.

Next, 10 ml of chloroform was injected to cool the obtained PET solution and wash the heating rod.

Next, the titration conditions were set according to the Mettler Toledo titration program, and titration of the cooled PET solution was carried out to calculate the content of carboxyl groups. Here, bromophenol blue was used as an indicator, and 0.1 N NaOH solution was used as a titrant.

Thereafter, the analysis procedure for carboxyl group content was repeated for 4 days to collect analysis data. The results are shown in Table 1 below.

### [Example 2]

The content of carboxyl groups was automatically analyzed through the same procedure as in Example 1, except that PET BR chips were used instead of PET semidal chips as the polymer sample. The results are shown in Table 1 below.

**[Table 1]**

| | | Blank (ml) | Sample amount (g) | Solvent amount (ml) | COOH (eq/ton) |
|---|---|---|---|---|---|
| PET semidal chip | Day 1 | 0.0574 | 0.1030 | 0.0794 | 21.36 |
| (COOH management content range: 27±1 eq/ton) | | | 0.1920 | 0.1026 | 23.55 |
| | Day 2 | 0.0577 | 0.1010 | 0.0807 | 22.81 |
| | | | 0.1020 | 0.0817 | 23.52 |
| | | | 0.1000 | 0.0805 | 22.77 |
| | Day 3 | 0.0552 | 0.1011 | 0.0807 | 25.28 |
| | | | 0.1020 | 0.0817 | 25.99 |
| | | | 0.1002 | 0.0805 | 25.24 |
| | Day 4 | 0.0526 | 0.1036 | 0.0753 | 21.89 |
| | | | 0.1037 | 0.0749 | 21.46 |
| | | | 0.1019 | 0.0742 | 21.17 |
| PET BR chip (COOH management content range: 56±2 eq/ton) | Day 1 | 0.0552 | 0.1001 | 0.1129 | 57.61 |
| | | | 0.1010 | 0.1147 | 58.96 |
| | | | 0.0994 | 0.1145 | 59.69 |
| | Day 2 | 0.0526 | 0.1087 | 0.1123 | 54.88 |
| | | | 0.1067 | 0.1118 | 55.48 |
| | | | 0.1030 | 0.1101 | 55.83 |
| PET semidal chip - raw materials: TPA and EG | | | | | |
| PET BR chip - raw materials: TPA and EG | | | | | |

Referring to Table 1 above, as a result of the automatic analysis of PET samples, it was confirmed that the content of carboxyl groups (COOH) was analyzed within the conventional management content range of carboxyl groups (COOH). This supports the fact that reliable analysis results can be obtained by analyzing the content of polymer end groups through the automatic analysis method (system) according to the present invention.

### [Example 3]

The content of carboxyl groups was automatically analyzed through the same procedure as in Example 1, except that the types of polymer samples were variously changed. The results are shown in Table 2 below.

**[Table 2]**

| Polymer sample type | COOH content according to automatic analysis (eq/ton) | Ref. COOH content (eq/ton) | COOH content deviation |
|---|---|---|---|
| ECOZEN^{®} | 24.6 | 24.3 | 101.3 |
| PETG | 6.6 | 5.8 | 113.5 |
| SKYBON^{®} | 7.8 | 7.4 | 105.2 |

Referring to Table 2 above, when the content of carboxyl groups (COOH) of various polymer samples was analyzed by the automatic analysis method (system) according to the present invention, it can be confirmed that the analysis was performed well within the range where the deviation in the content of carboxyl groups is not large.

### [Explanation of Reference Numerals]

| | | | |
|---|---|---|---|
| 10: | sample supply unit | | |
| 11: | auto sampler | 12: | container |
| 20: | heating unit | | |
| 21: | solvent injection tube | 22: | heating rod |
| 23: | cooling solvent injection tube | | |
| 30: | titration unit | | |
| 31: | indicator injection tube | 32: | titrant injection tube |
| 33: | colorimetric electrode | 34: | washing solvent injection tube |
| 40: | dispense control unit | | |
| 41: | solvent dispenser | 42: | indicator dispenser |
| 43: | titrant dispenser | | |
| 50: | pump control unit | | |
| 51: | solvent supply pump | 52: | cooling solvent supply pump |
| 53: | washing solvent supply pump | | |
| 60: | automatic data processing unit | | |
| 61: | software | 62: | output device |

## Claims

1. A method for automatic analysis of polymer end groups, which comprises:
(1) mounting a container containing a polymer sample into an auto sampler;
(2) adding a solvent comprising an aromatic compound having at least one hydroxy group into the container containing the polymer sample and heating it to obtain a polymer sample solution;
(3) cooling the polymer sample solution;
(4) adding an indicator to the cooled polymer sample solution and titrating it with a titrant to observe the change in the potential value of a colorimetric electrode; and
(5) analyzing the change in the potential value through software to calculate the content of polymer end groups.

2. The method for automatic analysis of polymer end groups of claim 1, wherein the aromatic compound in step (2) comprises at least one selected from the group consisting of phenol, o-cresol, m-cresol, p-cresol, o-chlorophenol, m-chlorophenol, p-chlorophenol, 2,6-xylenol, catechol, resorcinol, hydroquinone, pyrogallol, and benzyl alcohol.

3. The method for automatic analysis of polymer end groups of claim 1, wherein the solvent in step (2) further comprises at least one auxiliary solvent selected from the group consisting of chloroform, dichloromethane, and isopropyl alcohol.

4. The method for automatic analysis of polymer end groups of claim 3, wherein the mixing ratio of the aromatic compound to the auxiliary solvent is 5:1 to 1:5.

5. The method for automatic analysis of polymer end groups of claim 1, wherein the heating in step (2) is carried out at 60 to 100°C for 400 to 900 seconds.

6. The method for automatic analysis of polymer end groups of claim 1, wherein the cooling in step (3) is carried out by injecting at least one cooling solvent selected from the group consisting of chloroform, dichloromethane, and isopropyl alcohol into the polymer sample solution.

7. The method for automatic analysis of polymer end groups of claim 6, wherein the heating rod used to heat the polymer sample into which the solvent has been injected is washed by the cooling solvent in step (3).

8. The method for automatic analysis of polymer end groups of claim 1, wherein the indicator in step (4) comprises at least one selected from the group consisting of bromophenol blue, phenol red, and methyl red.

9. The method for automatic analysis of polymer end groups of claim 1, wherein the titrant in step (4) comprises at least one selected from the group consisting of sodium hydroxide (NaOH) and potassium hydroxide (KOH).

10. The method for automatic analysis of polymer end groups of claim 1, which further comprises (6) washing the colorimetric electrode of step (4) by supplying a washing solvent.

11. The method for automatic analysis of polymer end groups of claim 10, wherein the washing solvent is at least one selected from the group consisting of acetone, acetonitrile, isopropanol, and water.

12. The method for automatic analysis of polymer end groups of claim 1, wherein the polymer is a polymer having a carboxyl group.

13. The method for automatic analysis of polymer end groups of claim 1, wherein the end group is a carboxyl group (COOH).

14. A system for automatic analysis of polymer end groups, which comprises:
a sample supply unit comprising an auto sampler into which a container containing a polymer sample is mounted;
a heating unit comprising a solvent injection tube for injecting a solvent comprising an aromatic compound having at least one hydroxy group into the container containing the polymer sample, a heating rod for heating the polymer sample injected with the solvent, and a cooling solvent injection tube for injecting a cooling solvent to cool the polymer sample solution obtained through the heating;
a titration unit comprising an indicator injection tube for injecting an indicator into the polymer sample solution cooled through the cooling solvent, a titrant injection tube for injecting a titrant into the polymer sample solution in which the indicator has been injected, and a colorimetric electrode that shows a change in the potential value depending on the amount of the titrant injected;
a dispense control unit comprising a solvent dispenser for dispensing the solvent into the solvent injection tube, an indicator dispenser for dispensing the indicator into the indicator injection tube, and a titrant dispenser for dispensing the titrant into the titrant injection tube;
a pump control unit comprising a solvent supply pump for supplying the solvent to the solvent dispenser and a cooling solvent supply pump for supplying the cooling solvent to the cooling solvent injection tube; and
an automatic data processing unit comprising software for analyzing the change in the potential value and an output device for outputting analysis results obtained through the software.

15. The system for automatic analysis of polymer end groups of claim 14, wherein the container containing the polymer sample is a disposable container.

16. The system for automatic analysis of polymer end groups of claim 14, wherein the solvent further comprises at least one auxiliary solvent selected from the group consisting of chloroform, dichloromethane, and isopropyl alcohol.

17. The system for automatic analysis of polymer end groups of claim 14, wherein the colorimetric electrode is a photometric sensor electrode (optrode).

18. The system for automatic analysis of polymer end groups of claim 14, wherein the titration unit further comprises a washing solvent injection tube for injecting a washing solvent for washing the colorimetric electrode, and
the pump control unit further comprises a washing solvent supply pump that supplies the washing solvent to the washing solvent injection tube.
